# EUROPÄISCHE PATENTSCHRIFT

(11) **EP 2 247 241 B1**
(45) Veröffentlichungstag und Bekanntmachung des Hinweises auf die Patenterteilung: **21.03.2018**
(21) Anmeldenummer: 09715222.7
(22) Anmeldetag: 23.01.2009
(51) Int. Cl.: A61B 6/00, A61B 6/04

(54) **MEDIZINISCHES RÖNTGENGERÄT UND RÖNTGENSYSTEM**
MEDICAL RADIOLOGICAL DEVICE AND RADIOLOGICAL SYSTEM
APPAREIL RADIOGRAPHIQUE MÉDICAL ET SYSTÈME RADIOGRAPHIQUE MÉDICAL

(30) Priorität: 26.02.2008 DE 102008011157
(43) Veröffentlichungstag der Anmeldung: 10.11.2010
(73) Patentinhaber: Siemens Healthcare GmbH, 91052 Erlangen (DE)
(72) Erfinder: HÖRNIG, Mathias, 91052 Erlangen (DE)
(86) Internationale Anmeldenummer: PCT/EP2009/050740
(87) Internationale Veröffentlichungsnummer: WO 2009/106391

(56) Entgegenhaltungen:
- WO-A-2006/061357
- US-A- 5 930 328
- US-A1- 2007 232 881
- US-B1- 6 891 920

## Beschreibung

Die vorliegende Erfindung betrifft ein medizinisches Röntgensystem. Insbesondere betrifft die vorliegende Erfindung ein verbessertes medizinisches Röntgensystem mit einem Röntgengerät mit zumindest einem Element, das elektrisch verstellbar ist, um eine Anpassung des Röntgengeräts an Körpermaße eines zu untersuchenden Patienten zu ermöglichen.

Medizinische Röntgengeräte dienen zur Feststellung von Anomalien im Körper. Von einem Röntgenstrahler ausgesandte Röntgenstrahlung durchdringt dabei ein zu untersuchendes Körperteil und wird von einem elektronischen Sensor oder geeignetem Filmmaterial aufgenommen und anschließend ausgewertet. Dabei liegt das zu untersuchende Körperteil meist auf einem Tisch, welcher gleichzeitig den Sensor bzw. eine Filmkassette aufnimmt.

Häufig sind einige Elemente eines Röntgengeräts, etwa die Strahlenquelle und der Tisch, verstellbar, um eine Untersuchung verschieden großer Patienten mit dem Röntgengerät zu ermöglichen. Diese Einstellungen werden von einem Assistenten oder einem untersuchenden Arzt manuell vorgenommen. Während dieser Zeit ist das Gerät nicht anderweitig einsatzfähig, und auch der Assistent oder Arzt ist allein mit der Einstellung des Röntgengeräts beschäftigt. Hinzu tritt, daß Patienten häufig aufgeregt sind, wodurch die Einstellung zusätzlich erschwert wird.

Aus Patentschrift US 5,930,328 ist bekannt, die notwendigen Einstellungen anhand einer in einem Speicher des Röntgengeräts gespeicherten Patientengröße automatisch vorzunehmen. Die aus US 5,930,328 bekannte Lösung ist allerdings nur in Situationen vorteilhaft, in denen einerseits die Größe des Patienten aus früheren Untersuchungen bereits bekannt und andererseits auch im Speicher hinterlegt ist.

Aus Patentschrift WO2006/027341 ist bekannt, eine automatische Einstellung des Gerätes auf Basis von der durch ein im Röntgengerät eingebauten Meßsystem bestimmten Größe des Patienten.

Es ist daher eine Aufgabe der Erfindung, ein medizinisches Röntgensystem anzugeben, welches die Einschränkungen der Stand der Technik reduziert. Diese Aufgabe wird durch das in Anspruch 1 definierte medizinische Röntgensystem gelöst.

Vorteilhafte Ausgestaltungen der Erfindung sind in den Unteransprüchen angegeben.

Ein Vorteil der vorliegenden Erfindung ist darin zu sehen, daß ein erfindungsgemäßes Röntgensystem automatisch die Körpergröße eines Patienten erfaßt und anschließend ohne Zutun eines Assistenten oder Arztes das Röntgengerät automatisch passend zum jeweiligen Patienten eingestellt wird.

Im folgenden werden Ausführungsbeispiele der Erfindung anhand einer Zeichnung erläutert.

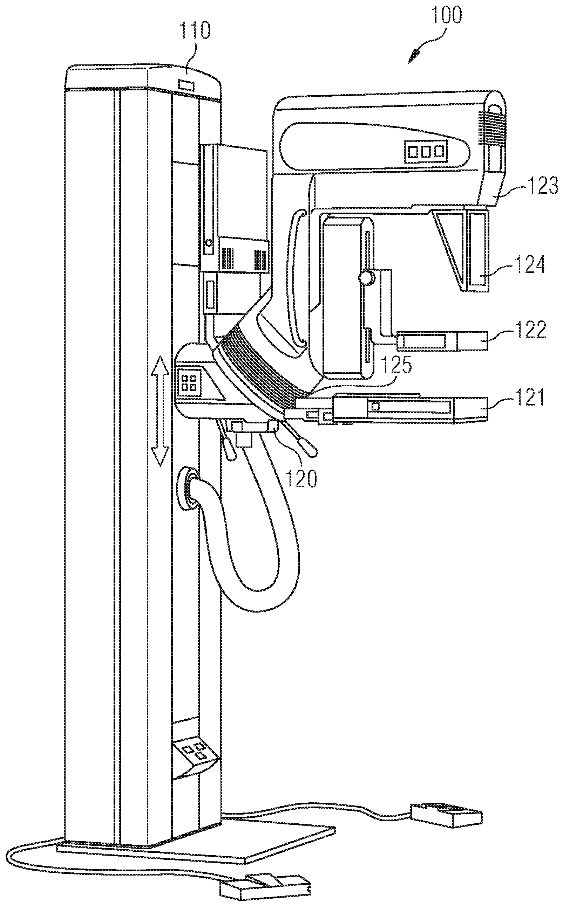

Die einzige Figur zeigt ein medizinisches Röntgengerät 100, wobei es sich beispielhaft um ein Mammographie-Röntgengerät handelt. Röntgengerät 100 weist ein feststehendes Ständerelement 110 auf, an dem ein Trägerelement 120 höhenverstellbar befestigt ist. Die Höhenverstellung des Trägerelements 120 erfolgt mittels eines elektrischen Motors im Ständerelement 110 und dient dazu, das Röntgengerät an verschiedene Körpergrößen von Patienten anzupassen. Dabei ist sowohl die Untersuchung stehender als auch sitzender Patienten möglich.

Trägerelement 120 trägt einen Tisch 121, welcher das zu untersuchende Körperteil stützt und einen digitalen Röntgensensor bzw. eine Filmkassette (nicht dargestellt) umfaßt. Eine ebenfalls am Trägerelement 120 befestigte optionale Platte 122 dient der Fixierung und ggf. Formung des zu untersuchenden Körperteils. Trägerelement 120 trägt schließlich eine Röntgenstrahlen erzeugende Apparatur 123 (im folgenden vereinfachend als Strahlenquelle bezeichnet). Dabei sind Tisch 121 und Strahlenquelle 123 so am Trägerelement 120 befestigt, daß sie in einer für die Bilderstellung geeigneten Weise zueinander ausgerichtet sind.

Trägerelement 120 bietet über ein Gelenk 125 die Möglichkeit, den gesamten Aufbau umfassend Tisch 121 und Strahlenquelle 123 motorgesteuert zu drehen, etwa um die durch Tisch 121 und Strahlenquelle 123 gebildete Strahlenachse um 45° zu drehen und so eine schräg-seitliche Aufnahme des zu untersuchenden Organs zu ermöglichen.

Beim dargestellten Röntgengerät 100 sind somit die Elemente Tisch 121 und Strahlenquelle 123 elektrisch verstellbar ausgeführt, um eine Anpassung des Röntgengeräts an verschiedene Körpergrößen von Patienten zu ermöglichen. Klassisch wird diese Anpassung durch einen Assistenten oder Arzt vorgenommen, während der Patient bereits unruhig vor dem Röntgengerät steht. Dies kostet Zeit und verstärkt des Patienten Unruhe, da die Verstellung aufgrund der Sensibilität und der Masse der Vorrichtung und der geforderten Präzision nur langsam vonstatten geht.
Erfindungsgemäß wird daher dem Röntgengerät über eine drahtgebundene oder drahtlose Schnittstelle (nicht dargestellt) ein Patientenparametersatz übermittelt, aus welchem eine Steuerung des Röntgengeräts (nicht dargstellt) eine gewünschte Einstellung der verstellbaren Elemente 121, 123 ermittelt und die entsprechenden Aktoren geeignet ansteuert, um diese gewünschte Position zu erhalten.
Der Patientenparametersatz umfaßt in einem Ausführungsbeispiel die Körpergröße des Patienten, welche in einem Vorraum durch ein vorzugsweise automatisches Meßsystem ermittelt wird. Damit ist eine Einstellung der Tischhöhe jedenfalls ungefähr möglich, so daß ggf. noch erforderliche Nachjustagen durch den für die Bildaufnahme ohnehin erforderlichen Assistenten oder Arzt rasch erledigt werden können.

Als automatisches Meßsystem kann beispielsweise ein System genutzt werden, welches mehrere, vorzugsweise äquidistant übereinander angeordnete Lichtschranken aufweist. Zur Messung eines Patienten stellt sich dieser in den Lichtschrankenbereich, und die Größe des Patienten wird aus den unterbrochenen Lichtschranken ermittelt.

Alternativ kann die Größe eines Patienten automatisch mittels einer Kamera ermittelt werden, wobei die Abbildung des Patienten programmgesteuert mit Referenzbildern verglichen wird, um die Größe des Patienten zu ermitteln. Bei der Verwendung einer Kamera können zudem andere Größenparameter ermittelt werden, für eine Mammographie-Untersuchung beispielsweise die Höhe und/oder Lager der Brust anstelle oder zusätzlich zur Größe des Patienten. Alternativ, und als nicht Teil der Erfindung, kann bei sich wiederholenden Untersuchungen eine einmal durch den Assistenten oder Arzt als optimal ermittelte Stellung der Elemente 121, 123 in die elektronische Patientenakte übernommen werden. Das Röntgengerät 100 weist hierfür Bedienmittel auf, mit welchen der Bediener dem Gerät mitteilt, daß die aktuelle Position die optimale Position ist. Daraufhin wird diese aktuelle Position (bzw. die diese Position charakterisierenden Parameter) von der Steuerung ermittelt, ein entsprechender Patientendatensatz daraus erzeugt, welcher wiederum drahtlos oder drahtgebunden an einen Rechner übertragen wird, welcher die elektronische Patientenakte speichert. Fortan kann bei erneuter Untersuchung des Patienten auf die optimalen Einstellungen zurückgegriffen werden, so daß nach Registrierung des Patienten für die Untersuchung das Röntgengerät 100 automatisch bereits die Elemente 121, 123 in die optimale Stellung bringt, während Patient und Assistent bzw. Arzt andere Untersuchungsschritte durchführen. Ebenfalls als nicht Teil der Erfindung, kann, falls die Größe des Patienten in der elektronische Patientenakte nicht vorhanden ist und vor der Röntgenuntersuchung nicht erfaßt werden kann, beispielsweise das Alter des Patienten für eine ungefähre Voreinstellung der verstellbaren Elemente 121, 123 herangezogen werden. Zusätzlich oder alternativ können weitere Besonderheiten Berücksichtigung finden, etwa daß eine Untersuchung im Sitzen erfolgt, beispielsweise weil der Patient nicht stehen kann.
Es sei darauf hingewiesen, daß fallweise neben der Höhe des Tischs 121 auch noch weitere Einstellungen vorgenommen werden müssen, etwa die Ausrichtung eines Schirms 124, die Stellung von verstellbaren Elementen zueinander (etwa der Abstand zwischen Strahlenquelle 123 und Tisch 121), usw. Diese zusätzlichen Parameter können ohne weiteres mit dem Patientenparametersatz in der elektronischen Patientenakte abgelegt werden.

Schließlich ist, insbesondere für größere medizinische Einrichtungen mit vielen Untersuchungsstationen, denkbar, die an einer ersten Untersuchungsstation gewonnenen Parameter und Einstellungen zur Voreinstellung der im Untersuchungsablauf folgenden (Röntgen-) Untersuchungsgeräte weiterzuverwenden. Dazu genügt es, daß an der ersten Untersuchungsstation geeignete Parameter ermittelt und in der elektronischen Patientenakte hinterlegt werden, die dann an den folgenden (Röntgen-) Untersuchungsgeräte zur Einstellung verstellbarer Elemente dienen.

## Patentansprüche

1. Medizinisches Röntgensystem, umfassend:
- ein medizinisches Röntgengerät (100) mit zumindest einem Element (121, 123), das elektrisch verstellbar ist, um eine Anpassung des Röntgengeräts (100) an Körpermaße eines zu untersuchenden Patienten zu ermöglichen, und einer Steuerung, welche folgendes aufweist:
- Schnittstellenmittel zum Empfangen eines Patientenparametersatzes, mit welchem Patientendaten verarbeitet und gespeichert werden;
- Mittel zum Berechnen einer Zielposition des Elements (121, 123) aus dem empfangenen Patientenparametersatz; und
- Mittel zum automatischen Steuern des elektrisch verstellbaren Elements (121, 123) in die Zielposition;
- ein automatisches Meßsystem, welches als Meßwerte zumindest die Körpergröße eines Patienten automatisch erfaßt; sowie
- einen Rechner, mit welchem Patientendaten verarbeitet und gespeichert werden und der folgendes aufweist:
- Mittel zum Erzeugen eines vorläufigen Patientenparametersatzes aus den Meßwerten; und
- Mittel zum Übertragen des vorläufigen Patientenparametersatzes sowie eines das automatische Steuern des elektrisch verstellbaren Elements (121, 123) in eine vorläufige Zielposition veranlassenden Steuerbefehls an das medizinische Röntgengerät,
**dadurch gekennzeichnet, dass**
das automatische Meßsystem sich einem Vorraum befindet.

2. Medizinisches Röntgensystem nach Anspruch 1, bei dem das automatische Meßsystem optisch arbeitet.

3. Medizinisches Röntgensystem nach einem der vorhergehenden Ansprüche, dessen elektrisch verstellbares Element (121, 123) eines der folgenden ist: ein Patiententisch, ein Detektortisch, ein Bucky Tray Tisch und/oder eine Strahlenquelle.

4. Medizinisches Röntgensystem, dessen Röntgengerät (100) ein Mammographie-Röntgengerät ist.

## Claims

1. Medical radiological system, comprising:
- a medical radiological device (100) having at least one element (121, 123) which is electrically adjustable, in order to enable an adaptation of the radiological device (100) to body dimensions of a patient to be examined, and a controller which comprises the following:
- interface means for receiving a patient parameter set with which patient data is processed and stored;
- means for calculating a target position of the element (121, 123) from the received patient parameter set; and
- means for automatically directing the electrically adjustable element (121, 123) into the target position;
- an automatic measuring system which automatically detects at least the body size of a patient as measured values; and
- a computer with which patient data is processed and stored and which comprises the following:
- means for generating a provisional patient parameter set from the measured values; and
- means for transmitting to the medical radiological device the provisional patient parameter set and a control command that triggers the automatic direction of the electrically adjustable element (121, 123) into a provisional target position,
**characterised in that**
the automatic measurement system is located in an anteroom.

2. Medical radiological system according to claim 1, in which the automatic measurement system operates optically.

3. Medical radiological system according to one of the preceding claims, the electrically adjustable element (121, 123) of which is one of the following: a patient table, a detector table, a Bucky tray table and/or a radiation source.

4. Medical radiological system, the radiological device (100) of which is a mammography radiological device.

## Revendications

1. Système radiographique médical, comprenant :
- un appareil (100) radiographique médical ayant au moins un élément (121, 123), réglable électriquement, pour rendre possible une adaptation de l'appareil (100) radiographique aux mensurations d'un patient à examiner et une commande, qui a ce qui suit :
- des moyens d'interface pour recevoir un jeu de paramètres du patient, par lesquels des données du patient sont traitées et mémorisées ;
- des moyens de calcul d'une position cible de l'élément (121, 123) à partir du jeu reçu de paramètres du patient et
- des moyens pour mettre automatiquement l'élément (121, 123) réglable électriquement dans la position cible ;
- un système automatique de mesure, qui détecte automatiquement, comme valeurs de mesure, au moins la taille d'un patient, ainsi que
- un ordinateur, par lequel les données du patient sont traitées et mémorisées et qui a ce qui suit :
- des moyens de production d'un jeu provisoire de paramètres du patient à partir des valeurs de mesure et
- des moyens de transmission à l'appareil radiographique médical du jeu provisoire de paramètres du patient, ainsi que d'une instruction de commande provoquant la mise automatique de l'élément (121, 123) réglable électriquement dans une position cible provisoire ;
**caractérisé en ce que** le système automatique de mesure se trouve dans une antichambre.

2. Système radiographique médical suivant la revendication 1, dans lequel le système automatique de mesure fonctionne de manière optique.

3. Système radiographique médical suivant l'une des revendications précédentes, dont l'élément (121, 123) réglable électriquement est l'un des suivants : une table pour patient, une table à détecteur, une table bucky tray et/ou une source de rayonnement.

4. Système radiographique médical, dont l'appareil (100) radiographique est un appareil radiographique de mammographie.
